(19) Europäisches Patentamt
European Patent Office
Office européen des brevets

(11) Publication number: 0 231 896 B1

# (12) EUROPEAN PATENT SPECIFICATION

(45) Date of publication of patent specification: 01.08.90

(51) Int. Cl.[5]: C 07 C 49/203, A 23 L 1/235, C 11 B 9/00

(21) Application number: 87101274.6

(22) Date of filing: 30.01.87

## (54) Alpha, beta-unsaturated ketones and their use as aroma chemicals.

(30) Priority: 31.01.86 JP 20946/86

(43) Date of publication of application: 12.08.87 Bulletin 87/33

(45) Publication of the grant of the patent: 01.08.90 Bulletin 90/31

(84) Designated Contracting States: BE CH DE FR GB IT LI NL

(56) References cited:
EP-A-0 037 584
EP-A-0 038 471
DE-A-2 431 039
DE-B-2 256 347
FR-A-2 242 358
GB-A-2 144 118

**The file contains technical information submitted after the application was filed and not included in this specification**

(73) Proprietor: KURARAY CO., LTD.
1621 Sakazu
Kurashiki-City Okayama Prefecture 710 (JP)

(72) Inventor: Kanehira, Koichi
2047-1, Aoeyama Sakazu
Kurashiki-City Okayama (JP)
Inventor: Fujita, Yoshiji
2-2-9, Showa
Kurashiki-City Okayama (JP)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 231 896 B1

## Description

### Field of the Invention

The present invention relates to α,β-unsaturated ketones, fragrance compositions containing said α,β-unsaturated ketones, and the use of said α,β-unsaturated ketones as aroma chemicals (fragrances) for imparting odors or flavors to substrate materials.

### Background of the Invention

It has been reported that 2-substituted-5-methyl-4-hexen-1-als such as 2,2,5-trimethyl-4-hexen-1-al, 2,5-dimethyl-2-ethyl-4-hexen-1-al, 2,5-dimethyl-2-n-propyl-4-hexen-1-al and 2,2-diethyl-5-methyl-4-hexen-1-al have sweet odors [cf. S. Watanabe et al., *Yukagaku* (Journal of the Japan Oil Chemist's Society), Vol. *31,* pages 295—299 (1982)].

It is desirable to provide aroma chemicals (fragrances) which have odors different from the odors of known aroma chemicals (fragrances; flavor materials), so that when such compounds are mixed with known aroma chemicals (fragrances) or finished fragrances, the resulting composition has a different odor note.

### Summary of the Invention

Accordingly, one object of the present invention is to provide α,β-unsaturated ketones, which have sweet odors and yet have odor notes which are different from the odor notes of known aroma chemicals (fragrances).

Another object of the present invention is to provide α,β-unsaturated ketones which are useful as aroma chemicals to be mixed with other aroma chemicals or perfumes. The invention furthermore relates to the use of said ketones as aroma chemicals for imparting odor or odor notes to a wide variety of substrate materials such as cosmetics and soaps or technical products.

Thus the present invention relates to α,β-unsaturated ketones of the general formula I

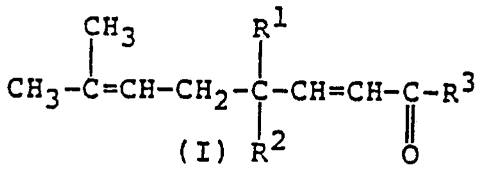

(I)

wherein $R^1$, $R^2$ and $R^3$ are the same or different and each represents a straight or branched lower alkyl group.

Furthermore, the present invention relates to fragrance compositions or perfumes which contain an olfactorily sensible amount of at least one of said α,β-unsaturated ketones.

The present invention also relates to the use of said α,β-unsaturated ketones as aroma chemicals (fragrances) for imparting an odor note to a substrate material.

### Detailed Description of the Preferred Embodiments

Referring to the general formula (I) above, each of the $R^1$, $R^2$ and $R^3$ substituents is a straight or branched lower alkyl group, preferably an alkyl group having from 1 to 4 carbon atoms such as methyl, ethyl, n-propyl, iso-propyl, n-butyl, isobutyl, sec-butyl or tert-butyl.

Examples of particularly preferred α,β-unsaturated ketones of the present invention are:

5,5,8-trimethyl-3,7-nonadien-2-one;
5,8-dimethyl-5-ethyl-3,7-nonadien-2-one;
5,8-dimethyl-5-(n-propyl)-3,7-nonadien-2-one;
5-(n-butyl)-5-ethyl-8-methyl-3,7-nonadien-2-one;
6,6,9-trimethyl-4,8-decadien-3-one;
2,6,6,9-tetramethyl-4,8-decadien-3-one;
7,7,10-trimethyl-5,9-undecadien-4-one;
8,8,11-trimethyl-6,10-dodecadien-5-one.

The α,β-unsaturated ketones of the present invention can be produced, for example by following synthetic route:

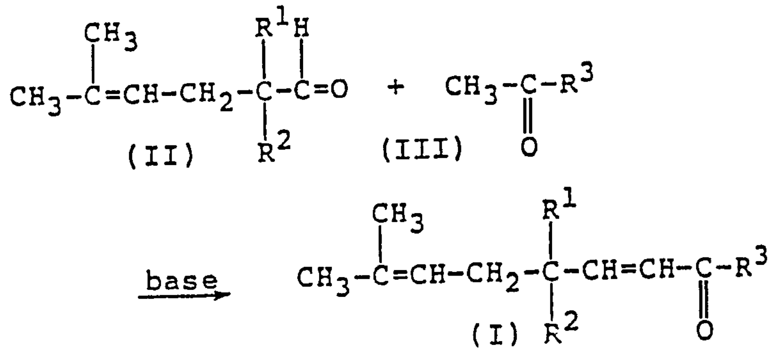


(II) (III) (I)

$R^1$, $R^2$ and $R^3$ are as defined above.

Thus, a 2-substituted-5-methyl-4-hexen-1-al of the general formula (II) is reacted with a ketone of the general formula (III) in the presence of a base to give an α,β-unsaturated ketone of the general formula (I). The ketone (III) is generally used in an approximately equimolar amount or excess amount relative to the 2-substituted-5-methyl-4-hexen-1-al (II). Examples of suitable bases include sodium hydroxide, potassium hydroxide, sodium methoxide, sodium ethoxide, potassium tert-butoxide and the like. The base is generally employed in a catalytic amount to an approximately equimolar amount relative to the ketone (III). The reaction is conducted in the presence or absence of a solvent. Examples of suitable solvents include methanol, ethanol, tert-butyl alcohol and water. The reaction may be conducted at temperatures ranging from about −70°C to about 150°C.

The α,β-unsaturated ketones of the general formula (I) have various floral aromas. By way of example 5,5,8-trimethyl-3,7-nonadien-2-one has a woody odor with an ozone-like note accompanied by a green top note, while 6,6,9-trimethyl-4,8-decadien-3-one has a floral oder accompanied by a sweet honey-like odor note. The α,β-unsaturated ketones of the general formula (I) have high diffusivity and retentivity and blend well with many other fragrances, and aroma chemicals and is thus of value as a raw material to be compounded with a fragrance or a fragrance composition which has a floral odor note such as muguet, rose, hyacinth or lilac.

The α,β-unsaturated ketones of the general formula (I) can be added to perfumes and fragrance compositions in a pure form or as a mixture to provide the desired odor note to a finished perfume.

The perfume and fragrance compositions obtained according to the present invention are suitable for use in a wide variety of perfumed articles and can also be used to enhance, modify or reinforce natural fragrant products.

The term "perfume and fragrance composition", as used herein, means a mixture of compounds including, for example, natural oils, synthetic oils, alcohols, aldehydes, ketones, esters, lactones, and frequently hydrocarbons which are admixed so that the combined odors of the individual components produce a pleasant or desired odor note. Such perfume and fragrance compositions usually contain (a) top notes which are usually low-boiling fresh-smelling fragrances, (b) a bouquet (middle note), (c) modifiers which round off and accompany the bouquet and, (d) end notes including fragrances which lend a particular note to the composition throughout all stages of evaporation and substances which retard evaporation.

Such perfume and fragrance compositions of the present invention can be used in conjunction with carriers, vehicles, solvents, dispersants, emulsifiers, surface-active agents, aerosol propellants and the like.

In perfume and fragrance compositions, the individual components contribute their particular olfactory characteristics to the composition, but the overall effect of the perfume and fragrance composition will be the sum of the effects of each ingredient. Thus, the α,β-unsaturated ketones of the general formula (I) can be used to alter the odor note of a perfume and fragrance composition, for example, by highlighting or moderating the olfactory reaction contributed by another ingredient of the composition.

The perfume and fragrance compositions according to the present invention contain an olfactorily sensible amount of an α,β-unsaturated ketone of the general formula (I), which varies according to the intended use of the composition; for example, it may range from about 0.005 weight percent to 95 weight percent. The perfume and fragrance composition of the present invention can be used in a large variety of ways. For example, it can be used as it is or in soaps; deodorants; perfumes and eau de Cologne; cosmetic preparations such as lotions, creams, etc; bath additives such as bath oil, bath salts, etc; hair preparations such as hair tonics, pomades, hair liquids, hair creams, stick pomades, shampoos, rinses, etc; cleansers; detergents and the like. In addition, the perfume and fragrance composition can be used for imparting an odor to technical products such as textile fibers and fabrics or paper products.

The α,β-unsaturated ketones of the general formula (I) are also useful as an ingredient for the preparation of artificial flavors and as flavor additives in foodstuffs, animal feeds, beverages, pharmaceutical preparations and tobacco products. The term "foodstuff" is used in this specification in its

broadest sense and is meant to include products such as coffee, tea and cocoa.

When the α,β-unsaturated ketones of the general formula (I) are used as a flavoring agent or an additive for modifying the organoleptic properties of foodstuffs, animal feeds, beverages, pharmaceutical preparations and tobacco products, the α,β-unsaturated ketones can be used in proportions ranging from 0.1 to 10 ppm, based on the weight of the products to be flavored. However, these proportions can be increased beyond 10 ppm up to about 100 ppm in order to achieve special flavoring effects. In the preparation of flavoring compositions by admixing an α,β-unsaturated ketone of the general formula (I) with other aroma chemicals, the same can be used, for example, in proportions of about 0.1% to about 15% of the total weight of the flavoring composition. In many cases, average proportions of about 1 to 10% by weight will give the desired results.

The above-mentioned fragrance compositions provided by this invention have a modern and high-quality oder which makes the best use of the odor of the α,β-unsaturated ketones of the general formula (I).

Having generally described this invention, a further understanding can be obtained by reference to certain specific examples which are provided herein for purposes of illustration only and are not intended to be limiting unless otherwise specified.

## Example 1

Synthesis of 5,5,8-trimethyl-3,7-nonadien-2-one

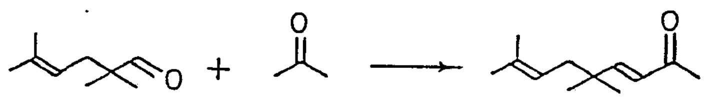

To a mixture of 250 g (1.79 mol) of 2,2,5-trimethyl-4-hexen-1-al and 750 g (12.9 mol) of acetone was added a solution of 36 g (0.643 mol) of potassium hydroxide in 1250 g of methanol and the whole mixture was stirred at 40—50°C for 23 hours. The reaction mixture was then cooled with ice and adjusted to a pH of about 4 with diluted hydrochloric acid, followed by addition of 10 g of sodium hydrogen carbonate. The reaction mixture was stirred for 2 hours, at the end of which time it was filtered and the filtrate was distilled under reduced pressure to remove a low-boiling fraction. The water was removed from the distillation residue and the remaining liquid was further subjected to vacuum distillation (51°C/226 Pa) to give 200 g (1.11 mol) of 5,5,8-trimethyl-3,7-nonadien-2-one as a colorless liquid. Yield 62%. The physical data of this product are as follows:

Mass spectrum (m/e): 180 [$M^+$]

$^1H$—NMR spectrum (90 MHz) $\delta_{HMS}^{CDCl_3}$: 6.70 (d, J=16Hz, 1H), 5.88 (d, J=16Hz, 1H), 5.15—4.87 (m, 1H), 2.16 (s, 3H), 1.95 (d, J=8Hz, 2H), 1.61 (s, 3H), 1.50 (s, 3H), 0.97 (s, 6H)

## Example 2

Synthesis of 6,6,9-trimethyl-4,8-decadien-3-one

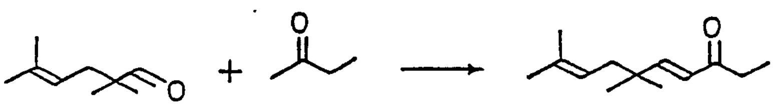

To a mixture of 230 g (1.64 mol) of 2,2,5-trimethyl-4-hexen-1-al and 1018 g (14.1 mol) of methyl ethyl ketone was added a solution of 33 g (0.59 mol) of potassium hydroxyde in 1150 g of methanol and the whole mixture was stirred at 40°C for 13.5 hours and, then, at 50°C for 27 hours. The reaction mixture was cooled with ice and adjusted to a pH of about 4 with diluted hydrochloric acid, followed by addition of 10 g of sodium hydrogen carbonate. The reaction mixture was stirred for 2 hours, at the end of which time it was filtered and the filtrate was distilled under reduced pressure to remove the low-boiling fraction. The water was removed from the residue and the remaining fluid was further subjected to vacuum distillation (80—82°C/266 Pa) to give 156 g (0.80 mol) of 6,6,9-trimethyl-4,8-decadien-3-one as a colorless liquid. Yield 49%. The physical data of this product are as follows:

Mass spectrum (m/e): 194 [$M^+$]

$^1H$—NMR spectrum (90 MHz) $\delta_{HMS}^{CDCl_3}$: 6.73 (d, J=16Hz, 1H), 5.91 (d, J=16Hz, 1H), 5.13—4.87 (m, 1H), 2.46 (q, J=7Hz, 2H), 1.94 (d, J=8Hz, 2H), 1.60 (s, 3H), 1.49 (s, 3H), 1.00 (t,) J=7Hz, 3H), 0.96 (s, 6H).

## Examples 3—5

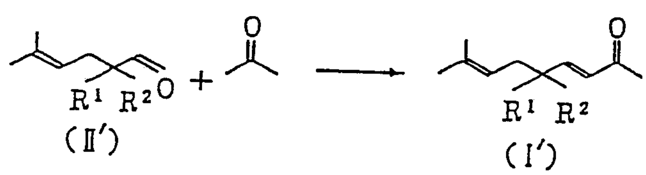

The reaction and work-up procedures of Example 1 were repeated except that various 2-substituted-5-methyl-4-hexen-1-als were used in lieu of 2,2,5-trimethyl-4-hexen-1-al to give the corresponding α,β-unsaturated ketone compounds. The yields and physical data of the products are shown in Table I.

TABLE I

| Example | 2-Substituted-5-methyl-4-hexen-1-al of formula (II') | | α,β-Unsaturated ketone of formula (I') | |
|---|---|---|---|---|
| | $R^1$ | $R^2$ | Yield (%) | Mass Spectrum (m/e) |
| 3 | $CH_3$ | $C_2H_5$ | 55 | 194 [$M^+$] |
| 4 | $CH_3$ | n—$C_3H_7$ | 52 | 208 [$M^+$] |
| 5 | $C_2H_5$ | n—$C_4H_9$ | 48 | 236 [$M^+$] |

Examples 6 and 7

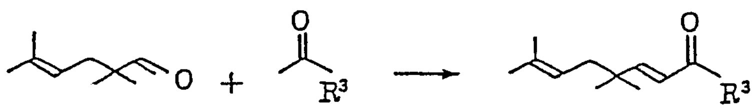

(III) (I'')

The reaction and work-up procedures of Example 2 were repeated except that various other ketones were used in lieu of methyl ethyl ketone to give the corresponding α,β-unsaturated ketone compounds. The yields and physical data of the products are shown in Table II.

TABLE II

| Example | Ketone of formula (III) | α,β-Unsaturated ketone of formula (I'') | |
|---|---|---|---|
| | $R^3$ | Yield (%) | Mass Spectrum (m/e) |
| 6 | i—$C_3H_7$ | 45 | 208 [$M^+$] |
| 7 | n—$C_4H_9$ | 46 | 222 [$M^+$] |

Example 8

Perfume and Fragrance Composition of the Muguet Type

A perfume and fragrance composition having a muguet-like odor was prepared from the following ingredients.

| | Parts by weight |
|---|---|
| Phenethyl alcohol | 15 |
| Hydroxycitronellal | 30 |
| Linalool | 5 |
| Benzyl alcohol | 5 |
| Benzyl salicylate | 3 |
| Geraniol | 5 |
| α-Terpineol | 5 |
| Linalyl acetate | 5 |
| Ylang ylang oil | 2 |
| Jasmin compound | 5 |
| Tetrahydrolinalcol | 2 |
| Heliotropine | 3 |
| Musk ketone | 2 |
| 5,5,8-Trimethyl-3,7-nonadien-2-one | 5 |
| | 92 |

Example 9

Perfume and Fragrance Composition of the White Rose Type

A perfume and fragrance composition having a white rose-like odor was prepared from the following ingredients.

| | Parts by weight |
|---|---|
| Geraniol | 20 |
| Benzyl acetate | 10 |
| Phenethyl alcohol | 10 |
| Rhodinol | 10 |
| Geranium oil | 5 |
| Phenethyl acetate | 5 |
| Bergamot oil | 5 |
| Linalool | 10 |
| Patchouli oil | 2 |
| Hydroxycitronellal | 5 |
| α-Ionone | 5 |
| Eugenol | 3 |
| Musk tincture | 5 |
| 5,5,8-Trimethyl-3,7-nonadien-2-one | 5 |
| | 100 |

Example 10

Perfume and Fragrance Composition of the Violet Type

A perfume and fragrance composition having a violet-like oder was prepared from the following ingredients.

| | Parts by weight |
|---|---|
| α-Ionone | 20 |
| Methylionone | 20 |
| Phenethyl alcohol | 10 |
| Benzyl alcohol | 5 |
| Linalcol | 5 |
| Bergamot oil | 3 |
| Cananga oil | 2 |
| Rose compound | 5 |
| Jasmin compound | 5 |
| Hydroxycitronellal | 5 |
| Violet leaf oil | 3 |
| Labdanum resin | 2 |
| Vetiver oil | 3 |
| Sandalwood oil | 2 |
| Musk ambrette | 5 |
| 5,5,8-Trimethyl-3,7-nonadien-2-one | 5 |
| | 100 |

Example 11

Perfume and Fragrance Composition of the Hyacinth Type

A perfume and fragrance composition having a hyacinth-like oder was prepared from the following ingredients:

| | Parts by weight |
|---|---|
| Phenethyl alcohol | 15 |
| Phenylacetaldehyde | 10 |
| Benzyl acetate | 5 |
| Methylionone | 5 |
| α-Ionone | 5 |
| Ylang ylang oil | 5 |
| Bergamot oil | 5 |
| Jasmin absolute | 3 |
| Rose absolute | 3 |
| Cinnamic alcohol | 10 |
| Dimethylbenzylcarbinol | 3 |
| Linalyl acetate | 5 |
| Musk tincture | 3 |
| 5,5,8-Trimethyl-3,7-nonadien-2-one | 5 |
| | 82 |

Example 12

Perfume and Fragrance Composition of the Modern Bouquet Type

A perfume and fragrance composition having a modern bouquet-like odor was prepared from the following ingredients:

| | Parts by weight |
|---|---|
| Phenethyl alcohol | 20 |
| Geraniol | 15 |
| Benzyl acetate | 15 |
| Methyl dihydrojasmonate | 10 |
| Rose compound | 5 |
| Jasmin compound | 5 |
| Bergamot oil | 5 |
| Methylionone | 5 |
| Aldehyde C-10 (10%) | 3 |
| Aldehyde C-11 (10%) | 3 |
| Sandalwood oil | 3 |
| Heliotropine | 3 |
| Musk ambrette | 5 |
| 6,6,9-Trimethyl-4,8-decadien-3-one | 5 |
| | 100 |

Example 13

Perfume and Fragrance Composition of the Lilac Type

A perfume and fragrance composition having a lilac-like odor was prepared from the following ingredients:

| | Parts by weight |
|---|---|
| α-Terpineol | 20 |
| Hydroxycitronellal | 15 |
| Linalool | 15 |
| Phenethyl alcohol | 15 |
| Benzyl alcohol | 5 |
| Ylang ylang oil | 5 |
| Geraniol | 5 |
| Cinnamic alcohol | 5 |
| Jasmin absolute | 2 |
| Styrax resin | 3 |
| Heliotropine | 5 |
| 6,6,9-Trimethyl-4,8-decadien-3-one | 5 |
| | 100 |

Example 14

Perfume and Fragrance Composition of the Jasmin Type

A perfume and fragrance composition having a jasmin-like odor was prepared from the following ingredients:

| | Parts by weight |
|---|---|
| α-Amylcinnamic aldehyde | 25 |
| Benzyl acetate | 30 |
| Hydroxycitronellal | 5 |
| Phenethyl alcohol | 5 |
| Linalyl acetate | 5 |
| Ylang ylang oil | 5 |
| Methylionone | 5 |
| Methyl dihydrojasmonate | 5 |
| Jasmin absolute | 2 |
| Isojasmone | 1 |
| Indole (1%) | 2 |
| Peru balsam | 2 |
| Heliotropine | 3 |
| 6,6,9-Trimethyl-4,8-decadien-3-one | 5 |
| | 100 |

Example 15

Perfume and Fragrance Composition of the Rose Type

A perfume and fragrance composition having a rose-like odor was prepared from the following ingredients:

| | Parts by weight |
|---|---|
| Phenethyl alcohol | 30 |
| Linalool | 5 |
| Linalyl acetate | 3 |
| Benzyl acetate | 10 |
| α-Ionone | 5 |
| Geraniol | 20 |
| Citronellol | 10 |
| Geranium oil | 5 |
| Eugenol | 1 |
| Rose absolute | 2 |
| Musk ambrette | 4 |
| 6,6,9-Trimethyl-4,8-decadien-3-one | 5 |
| | 100 |

Example 16

Plum Flavoring Composition

A plum flavoring composition for food use was prepared from the following ingredients:

| | Parts by weight |
|---|---|
| Ethyl acetate | 15 |
| Ethyl butyrate | 10 |
| Ethyl formate | 5 |
| Aldehyde C-16 | 4 |
| γ-Nonilactone | 2 |
| Benzaldehyde | 2 |
| Amyl alcohol | 7 |
| Amyl formate | 12 |
| Amyl butyrate | 15 |
| Allyl caproate | 1 |
| Citral | 1 |
| Orange oil | 10 |
| Clove oil | 2 |
| Coriander oil | 2 |
| Vanillin | 2 |
| 6,6,9-Trimethyl-4,8-decadien-3-one | 10 |
| | 100 |

Example 17

Raspberry Flavoring Composition

A raspberry flavoring composition for food use was prepared from the following ingredients:

| | Parts by weight |
|---|---|
| Ethyl acetate | 10 |
| Phenethyl alcohol | 10 |
| Amyl propionate | 5 |
| Geraniol | 5 |
| Benzyl acetate | 5 |
| Lemon oil | 10 |
| Isobutyl acetate | 5 |
| Clove oil | 2 |
| Hexyl acetate | 5 |
| Ethyl caproate | 3 |
| Merthylionone | 2 |
| Anisaldehyde | 3 |
| cis-3-Hexen-1-ol | 1 |
| Aldehyde C-9 | 3 |
| Aldehyde C-20 | 15 |
| Coumarin | 1 |
| 6,6,9-Trimethyl-4,8-decadien-3-one | 15 |
| | 100 |

**Claims**

1. An α,β-unsaturated ketone of the general formula I

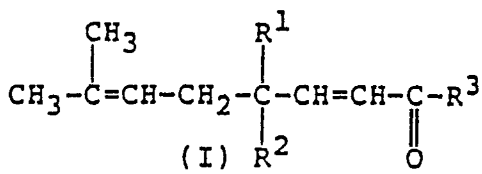

(I)

wherein $R^1$, $R^2$ and $R^3$ are the same or different and each represents a straight or branched lower alkyl group.

2. A fragrance or perfume composition, which comprises:
an olfactorily sensible amount of an α,β-unsaturated ketone of the general formula I

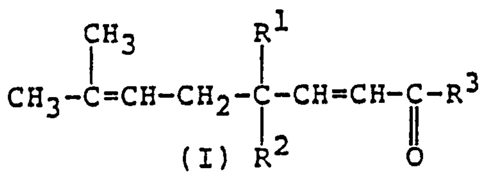

(I)

wherein $R^1$, $R^2$ and $R^3$ are the same or different and each represents a straight or branched lower alkyl group.

3. A process for imparting an aroma to a substrate material, comprising:
adding an olfactorily sensible amount of an α,β-unsaturated ketone of the general formula I

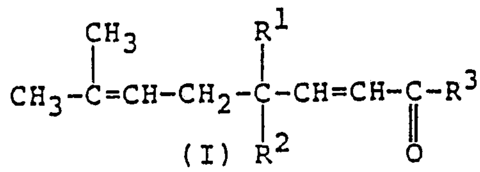

(I)

wherein $R^1$, $R^2$ and $R^3$ are the same or different and each represents a straight or branched lower alkyl group to the substrate material.

4. The use of an α,β-unsaturated ketone according to claim 1 as a fragrance or aroma chemical for imparting aromas to substrate materials.

**Patentansprüche**

1. α,β-ungesättigtes Keton der allgemeinen Formel I

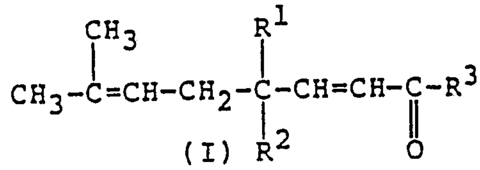

(I)

in der $R^1$, $R^2$ und $R^3$ gleich oder verschieden sind und jeweils einen unverzweigten oder verzweigten Niederalkylrest bedeuten.

2. Duft- oder Parfüm-Zusammensetzung, welche umfaßt:
eine geruchmäßig wahrnehmbare Menge eines α,β-ungesättigten Ketons der allgemeinen Formel I

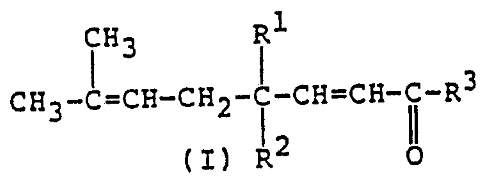

(I)

in der $R^1$, $R^2$ und $R^3$ gleich oder verschieden sind und jeweils einen unverzweigten oder verzweigten Niederalkylrest bedeuten.

3. Verfahren, einem Substratmaterial ein Aroma zu verleihen, umfassend:
Zugabe einer geruchsmäßig wahrnehmbaren Menge eines α,β-ungesättigten Ketons der allgemeinen Formel I

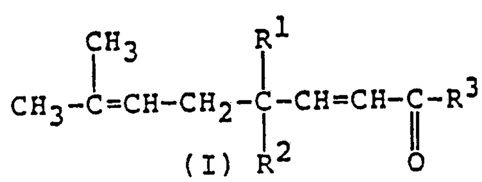

(I)

in der $R^1$, $R^2$ und $R^3$ gleich oder verschieden sind und jeweils einen unverzweigten oder verzweigten Niederalkylrest bedeuten, zu dem Substratmaterial.

4. Verwendung eines α,β-ungesättigten Ketons nach Anspruch 1 als Duft- oder Aroma-Chemikalie zur Verleihung von Aromata an Substratmaterialien.

**Revendications**

1. Une cétone α,β-insaturée répondant à la formule générale I

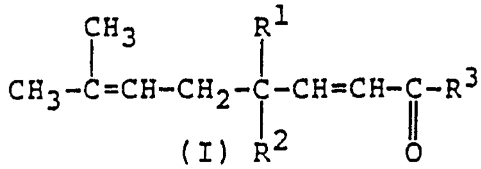

(I)

dans laquelle $R^1$, $R^2$ et $R^3$ sont semblables ou différents et représentent chacun groupe alcoyle inférieur droit ou ramifié.

2. Un parfum ou un composition odorante qui comprend une quantité sensible à l'olfaction d'une cétone α,β-insaturée répondant à la formule générale I.

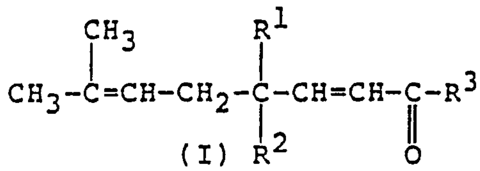

(I)

dans laquelle $R^1$, $R^2$ et $R^3$ sont semblables ou différents et représentent chacun un groupe alcoyle inférieur droit ou ramifié.

3. Un procédé pour conférer une arôme à un substrat comprenant l'addition d'une quantité sensible à l'olfaction d'une cétone α,β-insaturée répondant à la formule générale I

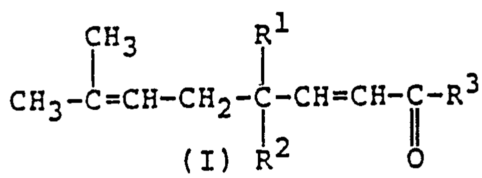

(I)

dans laquelle $R^1$, $R^2$ et $R^3$ sont semblables ou différents et représentent chacun un groupe alcoyle inférieur droit ou ramifié au substrat.

4. L'utilisation d'une cétone α,β-insaturée selon la revendication 1 comme parfum ou corps odorant pour conférer des arômes à des substrats.